# EUROPÄISCHE PATENTSCHRIFT

(11) **EP 0 584 388 B1**
(45) Veröffentlichungstag und Bekanntmachung des Hinweises auf die Patenterteilung: **08.07.1998**
(21) Anmeldenummer: 92114546.2
(22) Anmeldetag: 26.08.1992
(51) Int. Cl.: A61N 1/37, A61N 1/365, A61B 5/08, A61B 5/091

(54) **Herzschrittmacher zum Erzeugen eines dem Atemzeitvolumen eines Patienten entsprechenden Signals**
Pacemaker for producing a signal corresponding to a patient's minute-volume
Stimulateur cardiaque de production d'un signal correspondant au volume-par-minute d'un patient

(43) Veröffentlichungstag der Anmeldung: 02.03.1994
(73) Patentinhaber: Pacesetter AB, 171 95 Solna (SE)
(72) Erfinder: Sivard, Ake, S-171 55 Solna (SE)
(74) Vertreter: Lettström, Richard Wilhelm

(56) Entgegenhaltungen:
- EP-A- 0 135 911
- EP-A- 0 310 025
- EP-A- 0 327 292
- US-A- 4 733 667
- MEDICAL AND BIOLOGICAL ENGINEERING AND COMPUTING. Bd. 20, Nr. 3, Mai 1982, STEVENAGE GB Seiten 293 - 298 A.J.WILSON ET AL. 'Methods of filtering the heart-beat artefact from the breathing waveform of infants obtained by impedance pneumography'

## Beschreibung

Herzschrittmacher zum Erzeugen eines dem Atemzeitvolumen eines Patienten entsprechenden Signals, wobei mit Hilfe einer im Bereich des Herzens angeordneten Elektrodenanordnung und einer daran angeschlossenen Impedanzmeßeinrichtung ein Impedanzmeßsignal erfaßt wird, mittels einer Filteranordnung eine mit der Atmung des Patienten korrelierende Signalkomponente aus dem Impedanzmeßsignal herausgefiltert wird, mittels eines Nulldurchgangdetektors Nulldurchgänge der herausgefilterten Signalkomponente detektiert werden und bei jedem Nulldurchgang eine vom Betrag der Signalkomponente abhängige Größe erfaßt wird, die zur laufenden Bildung eines dem Atemzeitvolumen entsprechenden Mittelwerts während eines vorgegebenen Zeitintervalls herangezogen wird.

Bei einem derartigen, aus der US-A-4 901 725 bekannten Herzschrittmacher wird mittels der Elektrodenanordnung eines Herzschrittmachers und einer Impedanzmeßeinrichtung im Herzschrittmacher ein Impedanzmeßsignal vom Herzen eines Patienten abgeleitet, das sich sowohl in Abhängigkeit von der Pumptätigkeit des Herzens als auch von außen auf das Herz einwirkenden, durch die Atmung und durch Bewegungen des Patienten hervorgerufenen, intrathorakalen Druckschwankungen ändert. Durch Bandpaßfilterung wird aus dem Impedanzmeßsignal eine mit der Atmung korrelierende Signalkomponente herausgefiltert, indem Frequenzanteile des Impedanzmeßsignals unter 0,05 Hz und über 0,8 Hz unterdrückt werden. Die so erhaltene Signalkomponente wird abgetastet und sowohl einem Nulldurchgangdetektor als auch einem Betragsmittelwertbildner zugeführt, der die Beträge der Abtastwerte über eine wenigen Atemzügen entsprechende Dauer zu einem Betragsmittelwert der Signalkomponente mittelt. Dabei wird davon ausgegangen, daß der Betragsmittelwert der Signalkomponente dem mittleren Atemzugvolumen entspricht. Bei jedem detektierten Nulldurchgang im Verlauf der Signalkomponente wird der momentane Betragsmittelwert der Signalkomponente einem weiteren Mittelwertbildner zugeführt. Dieser erzeugt einen Mittelwert, in den der bei jedem Nulldurchgang erneut bestimmte Betragsmittelwert der Signalkomponente, welcher dem mittleren Atemzugvolumen entspricht, entsprechend der Häufigkeit der Nulldurchgänge, welche die Atemfrequenz bezeichnen, eingeht, so daß der Mittelwert dem Produkt aus Atemzugvolumen und Atemfrequenz und damit dem Atemzeitvolumen entspricht. Das so ermittelte Atemzeitvolumen wird zur Frequenzsteuerung des Herzschrittmachers herangezogen.

Wie bereits erwähnt, wird bei dem bekannten Herzschrittmacher davon ausgegangen, daß der Betragsmittelwert der aus dem Impedanzmeßsignal herausgefilterten und mit der Atmung korrelierenden Signalkomponente dem mittleren Atemzugvolumen entspricht. Eine solche Annahme trifft aber nur dann zu, wenn der Verlauf der Signalkomponente zumindest annähernd sinusförmig ist und symmetrisch zur Nullinie liegt. In der Praxis sind aber der Signalkomponete trotz der Filterung Signalanteile überlagert, die auf der Herztätigkeit und Bewegungen des Patienten beruhen. Solche Signalanteile sind zwar auf Grund der Filterung in ihrer Signalhöhe reduziert, können aber Nulldurchgänge im Verlauf der Signalkomponente hervorrufen, die als des Impedanzmeßsignals Ein- oder Ausatmung detektiert werden und bei dem bekannten Verfahren mit dem ermittelten Wert für das mittlere Atemzugvolumen bewertet werden. Eine weitere Fehlerquelle besteht darin, daß die untere Grenzfrequenz für die Bandpaßfilterung des Impedanzmeßsignals im Hinblick auf die niedrigste zu erwartende Atemfrequenz auf einen sehr niedrigen Wert, bei dem bekannten Verfahren 0,05 Hz festgesetzt ist, so daß die Nullinie der Signalkomponente während der nur einigen wenigen Atemzügen entsprechenden Dauer für die Bildung des Betragsmittelwerts der Signalkomponente erheblich schwanken kann und nicht erfaßbar ist. Die Bildung der Beträge der Signalkomponente und deren Mittelung erfolgt daher ausgehend von einer fiktiven Nullinie, wobei die Abweichungen der fiktiven Nullinie von der realen Nullinie zu einem Gleichanteil bei der Mittelwertbildung führen, der sich als Fehler bei der Bestimmung des Atemzugvolumens auswirkt.

Der Erfindung liegt die Aufgabe zugrunde, auf eine möglichst einfache Weise eine hinreichend genaue Bestimmung des Atemzeitvolumens zu ermöglichen.

Gemäß der Erfindung wird die Aufgabe dadurch gelöst, daß bei dem Herzschrittmacher der eingangs angegebenen Art als vom Betrag der Signalkomponente abhängige Größe der Maximalwert des Betrags der Signalkomponente zwischen jeweils zwei aufeinanderfolgenden Nulldurchgängen erfaßt wird. Die Summe aus jeweils zwei nacheinander erfaßten Maximalwerten des Betrags der Signalkomponente zwischen jeweils zwei Nulldurchgängen der Signalkomponente entspricht der Schwankungshöhe der mit der Atmung korrelierenden Signalkomponente und damit dem Atemzugvolumen eines Atemzugs. Dabei ist die für die Bestimmung der Beträge der Signalkomponente gewählte Lage der Nullinie ohne Bedeutung für das Ergebnis der Bestimmung des Atemzugvolumens, weil eine Änderung der Lage der Nullinie dazu führt, daß der eine Maximalwert in dem Maße vergrößert wird, wie der andere Maximalwert verringert wird, so daß die Summe aus den beiden Maximalwerten und damit der Wert für das Atemzugvolumen konstant bleibt.

Soweit der mit der Atmung korrelierenden Signalkomponente des Impedanzmeßsignals andere Signalanteile überlagert sind, die nicht von der Atmung sondern von der Herztätigkeit oder der Bewegung des Patienten herrühren, so ist die Signalhöhe dieser Signalanteile durch die Filterung des Impedanzmeßsignals bereits erheblich reduziert und beeinflußt daher die Bestimmung des Atemzeitvolumens entsprechend dem erfindungsgemäßen Herzschrittmacher nur in geringem Maße. Wenn allerdings diese Signalanteile die Signalkomponente im Bereich der Nullinie stören, kann dies zu zusätzlichen Detektionen von Nulldurchgängen führen; da entsprechend dem erfindungsgemäßen Herzschrittmacher zur Bestimmung des Atemzeitvolumens die Maximalwerte der Signalkomponenete zwischen jeweils zwei aufeinanderfolgenden Nulldurchgängen herangezogen werden und die störenden Signalanteile bezüglich ihrer Amplitude bereits erheblich reduziert sind, ist der dadurch verursachte Fehler bei der Bestimmung des Atemzeitvolumens nur gering.

Entsprechend einer vorteilhaften Weiterbildung des erfindungsgemäßen Herzschrittmachers ist vorgesehen, daß zur Bildung des Maximalwerts des Betrags der Signalkomponente zwischen zwei Nulldurchgängen Abtastwerte des Betrags der Signalkomponente einem ersten Eingang eines Komparators zugeführt werden, dessen zweiten Eingang der Inhalt einer Speichereinrichtung zugeführt wird, und daß der aktuelle Inhalt der Speichereinrichtung durch den aktuellen Abtastwert des Betrags der Signalkomponente ersetzt wird, wenn der Betrag der Signalkomponente den Wert des Speicherinhalts übersteigt.

Die Bildung des dem Atemzeitvolumen entsprechenden Mittelwerts erfolgt auf einfachste Weise dadurch, daß die während des vorgegebenen Zeitintervalls erfaßten Maximalwerte in einem Addierer aufsummiert werden.

Zur Erläuterung der Erfindung wird im folgenden auf die Figuren der Zeichnung Bezug genommen; im einzelnen zeigen
- FIG 1: ein Blockschaltbild eines frequenzgesteuerten Herzschrittmachers, der eine Einrichtung zur Bestimmung des Atemzeitvolumens aus einem vom Herzen abgeleiteten Impedanzmeßsignal aufweist,
- FIG 2: ein Blockschaltbild der in Figur 1 gezeigten Einrichtung zur Bestimmung des Atemzeitvolumens und
- FIG 3: ein Beispiel für den Verlauf einer aus dem Impedanzmeßsignal herausgefilterten und mit der Atmung korrelierenden Signalkomponente.

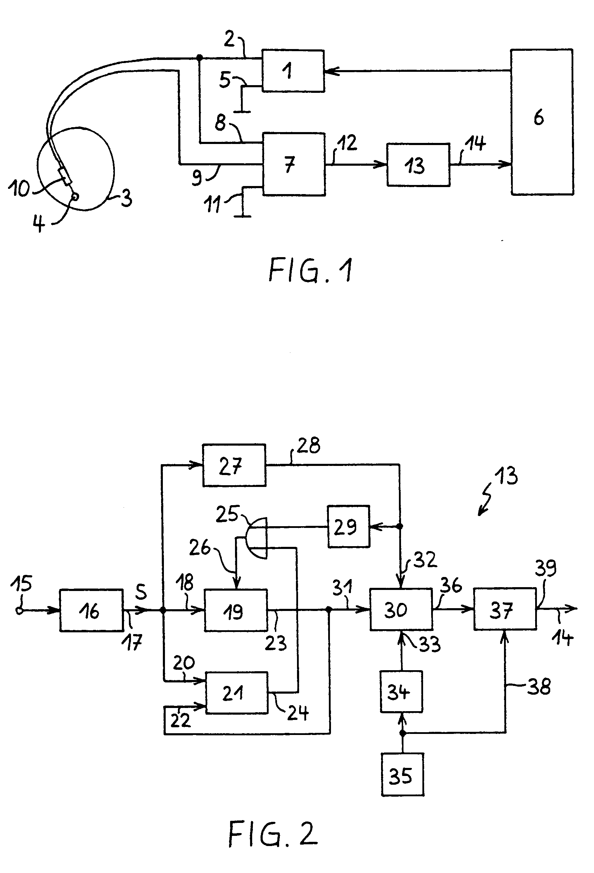

Figur 1 zeigt das Blockschaltbild eines Herzschrittmachers mit einem Stimulationsimpulsgenerator 1, der an einem ersten Ausgangsanschluß 2 mit einer im Herzen 3 eines Patienten angeordneten Stimulationselektrode 4 und an einem zweiten Ausgangsanschluß 5 mit einem als Gegenelektrode zur Stimulationselektrode 4 dienenden Gehäuse des Herzschrittmachers verbunden ist. Zur Steuerung der Stimulationsimpulsabgabe ist der Stimulationsimpulsgenerator 1 an einer Herzschrittmachersteuerung 6 angeschlossen. Eine Impedanzmeßeinrichtung 7 ist über einen Meßstromausgang 8 mit der Stimulationselektrode 4, über einen Spannungsmeßeingang 9 mit einer von der Stimulationselektrode 4 beabstandeten Ringelektrode 10 und an einem Bezugsanschluß 11 mit dem Gehäuse des Herzschrittmachers verbunden. Die Impedanzmeßeinrichtung 7 erzeugt zu vorgegebenen Zeitpunkten zwischen der Stimulationselektrode 4 und dem Gehäuse des Herzschrittmachers einen Meßstrom, wobei der von dem Meßstrom im Bereich des Herzens 3 zwischen der Stimulationselektrode 4 und der Ringelektrode 10 oder der Ringelektrode 10 und dem Gehäuse des Herzschrittmachers hervorgerufene Spannungsabfall von der Impedanzmeßeinrichtung 7 gemessen wird. Die Impedanzmeßeinrichtung 7 erzeugt an ihrem Ausgang 12 ein Impedanzmeßsignal, das einer Einrichtung 13 zur Bestimmung des Atemzeitvolumens aus dem Impedanzmeßsignal zugeführt wird. Die Einrichtung 13 ist ausgangsseitig über eine Steuerleitung 14 an der Herzschrittmachersteuerung 6 angeschlossen, die die Stimulationsimpulsabgabe in Abhängigkeit von dem erfaßten Atemzeitvolumen steuert.

Figur 2 zeigt ein Blockschaltbild der Einrichtung 13 zur Ermittlung des Atemzeitvolumens aus dem Impedanzmeßsignal. Die Einrichtung 13 weist einen mit dem Impedanzmeßsignal beaufschlagten Eingang 15 auf, an dem eine Filteranordnung 16 angeschlossen ist. Die Filteranordnung 16 besteht im wesentlichen aus einem Bandpaßfilter, das einen Durchlaßbereich von etwa 0,1 bis 0,7 Hz aufweist und somit die nicht mit der Atmung korrelierenden, insbesondere von der Herztätigkeit und der Bewegung des Patienten verursachten Signalanteile des Impedanzmeßsignals unterdrückt. Diese Signalunterdrückung erfolgt insoweit unvollkommen, als sehr niedrige Herzschlagfrequenzen oder die Frequenzen bestimmter rhythmischer Bewegungen des Patienten in den für die Atmung des Patienten charakteristischen Frequenzbereich fallen können. Um den Frequenzdurchlaßbereich des Bandpaßfilters so schmal wie möglich einstellen zu können, kann vorgesehen sein, daß die Mittenfrequenz des Bandpaßfilters variabel ist und an die momentane Atemfrequenz des Patienten angepaßt ist. Die Filteranordnung 16 ist ferner vorzugsweise als Digitalfilter ausgebildet, so daß am Ausgang 17 der Filteranordnung 16 eine mit der Atmung korrelierende Signalkomponente S in Form von digitalisierten Abtastwerten vorliegt, der in begrenztem Umfang von der Herztätigkeit und der Bewegung des Patienten hervorgerufene Signalanteile überlagert sind.

Die Filteranordnung 16 ist an ihrem Ausgang 17 mit dem Dateneingang 18 eines Speicherregisters 19 und einem ersten Eingang 20 eines Komparators 21 verbunden, dessen zweiter Eingang 22 an einem Datenausgang 23 des Speicherregisters 19 angeschlossen ist. Wenn der dem ersten Eingang 20 des Komparators 21 zugeführte Abtastwert der Signalkomponente S den an dem zweiten Eingang 22 des Komparators 21 anliegenden Wert des Inhalts des Speicherregisters 19 überschreitet, erzeugt der Komparator 21 an seinem Ausgang 24 ein Ausgangssignal, das über ein ODER-Glied 25 einem Steuereingang 26 des Speicherregisters 19 zur Übernahme des an dem Dateneingang 18 anliegenden Abtastwerts der Signalkomponente S zugeführt wird.

An dem Ausgang 17 der Filteranordnung 16 ist schließlich ein Nulldurchgangdetektor 27 angeschlossen, der jeden Vorzeichenwechsel bei den digitalen Abtastwerten der Signalkomponente S detektiert und dabei an seinem Ausgang 28 ein Steuersignal erzeugt. Um zu verhindern, daß von Rauschsignalanteilen der Signalkomponente S hervorgerufene Vorzeichenwechsel fälschlicherweise als Nulldurchgänge der Signalkomponente S detektiert werden, erfolgt die Detektion von Vorzeichenwechseln vorzugsweise mit einer Hysterese. Der Ausgang 28 des Nulldurchgangdetektors 27 ist über ein Verzögerungsglied 29 und das ODER-Glied 25 mit dem Steuereingang 26 des Speicherregisters 19 verbunden.

Ein Addierer 30 ist mit einem Dateneingang 31 an dem Datenausgang 23 des Speicherregisters 19 angeschlossen und an einem Steuereingang 32 mit dem Ausgang 28 des Nulldurchgangdetektors 27 verbunden. Jedesmal dann, wenn der Nulldurchgangdetektor 27 ein Ausgangssignal erzeugt, wird der aktuelle Speicherinhalt des Speicherregisters 19 von dem Addierer 30 übernommen und zu dem in ihm enthaltenen Summenwert hinzuaddiert. Dabei entspricht der übernommene Speicherinhalt dem maximalen Abtastwert der Signalkomponente S zwischen dem soeben detektierten Nulldurchgang und dem davor detektierten Nulldurchgang. Der Addierer 30 weist ferner einen Rücksetzeingang 33 auf, an dem über ein weiteres Verzögerungsglied 34 eine Zeitgeberschaltung 35 angeschlossen ist, die alle fünf Sekunden einen Ausgangsimpuls erzeugt. An dem Ausgang 36 des Addierers 30 ist ein Mittelwertbildner 37 in Form eines Schieberegisters angeschlossen, das über eine Steuerleitung 38 mit der Zeitgeberschaltung 35 verbunden ist. Alle fünf Sekunden wird der Summenwert am Ausgang 36 des Addierers 30 in das Schieberegister 37 übernommen und zu einer vorgegebenen Anzahl von beispielsweise fünf jeweils zuletzt übernommenen Summenwerten hinzuaddiert. Auf diese Weise wird in dem Schieberegister 37 ein laufender Mittelwert des Atemzeitvolumens wahrend der letzten 30 Sekunden gebildet, der am Ausgang 39 des Mittelwertbildners 37 über die Steuerleitung 14 der Herzschrittmachersteuerung 6 (Fig. 1) zugeführt wird.

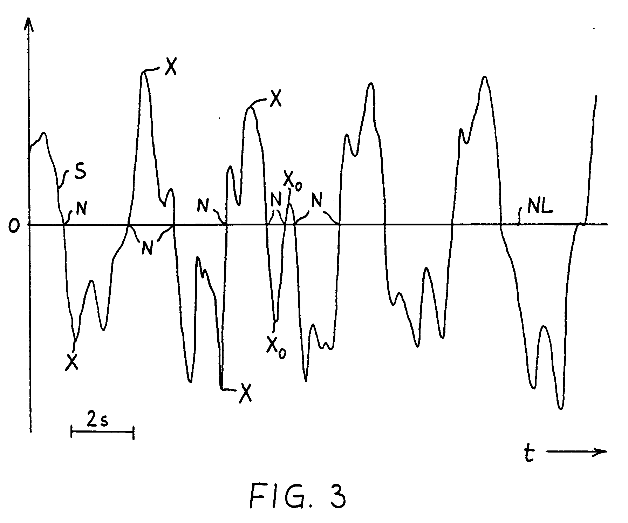

Wie Figur 3 zeigt, weicht der Verlauf der aus dem Impedanzsignal herausgefilterten Signalkomponente S auf Grund von Signalanteilen, die auf Bewegungsartefakten mit Frequenzen im Bereich der Atemfrequenz beruhen, von einem idealen sinusförmigen Verlauf ab. In der Einrichtung 13 nach Figur 2 werden die Nulldurchgänge N der Signalkomponente S in bezug auf eine Nullinie NL detektiert und der betragsmäßig größte Wert X der Signalkomponente S zwischen jeweils zwei aufeinanderfolgenden Nulldurchgängen N ermittelt. Die Summe von zwei nacheinander ermittelten Maximalwerten X entspricht dabei dem Atemzugvolumen eines Atemzugs und die während der vorgegebenen Zeitdauer von fünf Sekunden in dem Addierer 30 aufsummierten Maximalwerte X ergeben ein Maß für das Atemzeitvolumen.

Wie Figur 3 ferner zeigt, können auch nicht mit der Atmung in Zusammenhang stehende Extremwerte X₀ in die Bestimmung des Atemzeitvolumens eingehen, jedoch ist der Einfluß solcher Störungen auf die Bestimmung des Atemzeitvolumens aufgrund ihres seltenen Auftretens und der Vorfilterung nur gering.

### Bezugszeichenliste

- 1: Stimulationsimpulsgenerator
- 2: erster Ausgangsanschluß von 1
- 3: Herz
- 4: Stimulationselektrode
- 5: zweiter Ausgangsanschluß von 1
- 6: Herzschrittmachersteuerung
- 7: Impedanzmeßeinrichtung
- 8: Meßstromausgang
- 9: Spannungsmeßeingang
- 10: Ringelektrode
- 11: Bezugsanschluß
- 12: Ausgang von 7
- 13: Einrichtung zur Bestimmung des Atemzeitvolumens
- 14: Steuerleitung
- 15: Eingang von 13
- 16: Filteranordnung
- 17: Ausgang von 16
- 18: Dateneingang von 19
- 19: Speicherregister (Speichereinrichtung)
- 20: erster Eingang von 21
- 21: Komparator
- 22: zweiter Eingang von 21
- 23: Datenausgang von 19
- 24: Ausgang von 21
- 25: ODER-Glied
- 26: Steuereingang von 19
- 27: Nulldurchgangdetektor
- 28: Ausgang von 27
- 29: Verzögerungsglied
- 30: Addierer
- 31: Dateneingang von 30
- 32: Steuereingang von 30
- 33: Rücksetzeingang von 30
- 34: weiteres Verzögerungsglied
- 35: Zeitgeberschaltung
- 36: Ausgang von 30
- 37: Mittelwertbildner (Schieberegister)
- 38: Steuerleitung
- 39: Ausgang von 37
- N: Nulldurchgänge von S
- NL: Nullinie
- S: Signalkomponente
- X: Maximalwerte von S
- X₀: nicht in Zusammenhang mit der Atmung stehende Maximalwerte von S

## Patentansprüche

1. Herzschrittmacher zum Erzeugen eines dem Atemzeitvolumen eines Patienten entsprechenden Signals, wobei mit Hilfe einer im Bereich des Herzens (3) angeordneten Elektrodenanordnung (4,10) und einer angeschlossenen Impedanzmeßeinrichtung (7) ein Impedanzmeßsignal erfaßt wird, mittels einer Filteranordnung (16) eine mit der Atmung des Patienten korrelierende Signalkomponente (S) aus dem Impedanzsignal herausgefiltert wird, mittels eines Nulldurchgangdetektors (27) Nulldurchgänge (N) der herausgefilterten Signalkomponente (S) detektiert werden und bei jedem Nulldurchgang (N) eine vom Betrag der Signalkomponente (S) abhängige Größe erfaßt wird, die zur laufenden Bildung eines dem Atemzeitvolumen entsprechenden Mittelwerts während eines vorgegebenen Zeitintervalls herangezogen wird, **da-durch gekennzeichnet**, daß als vom Betrag der Signalkomponente (S) abhängige Größe der betragsmässig grösste Wert (X) des Betrags der Signalkomponente (S) zwischen jeweils zwei aufeinanderfolgenden Nulldurchgängen (N) erfaßt wird, und dass die Summe aus jeweils zwei nacheinander erfassten grössten Werten gebildet wird.

2. Herzschrittmacher nach Anspruch 1, **dadurch gekennzeichnet**, daß zur Bildung des Maximalwerts (X) des Betrags der Signalkomponente (S) zwischen zwei Nulldurchgängen (N) Abtastwerte des Betrags der Signalkomponente (S) einem ersten Eingang (20) eines Komparators (21) zugeführt werden, dessen zweiten Eingang (22) der Inhalt einer Speichereinrichtung (19) zugeführt wird, und daß der jeweils aktuelle Inhalt der Speichereinrichtung (19) durch den aktuellen Abtastwert des Betrags der Signalkomponente (S) ersetzt wird, wenn der Betrag der Signalkomponente (S) den Wert des Speicherinhalts übersteigt.

3. Herzschrittmacher nach Anspruch 1 oder 2, **dadurch gekennzeichnet**, daß zur Bildung des Mittelwerts die während des vorgegebenen Zeitintervalls erfaßten Maximalwerte (X) in einem Addierer (30) aufsummiert werden.

## Claims

1. Heart pacemaker for generating a signal which corresponds to the respiratory period volume of a patient, with an impedance measurement signal being determined with the aid of an electrode arrangement (4, 10), which is arranged in the region of the heart (3), and a connected impedance measuring device (7), a signal component (S) which correlates with the breathing of the patient being filtered out of the impedance signal by means of a filter arrangement (16), zero crossings (N) of the signal component (S) which has been filtered out being detected by means of a zero crossing detector (27), and a variable which is dependent on the amount of the signal component (S) being determined at each zero crossing (N), which variable is used during a specified time interval for the running formation of a mean value which corresponds to the respiratory period volume, characterised in that there is determined as a variable which is dependent on the amount of the signal component (S) the value (X) of the amount of the signal component (S) between each two successive zero crossings (N) that is the greatest in terms of amount, and in that the sum of each two successively determined greatest values is formed.

2. Heart pacemaker according to claim 1, characterised in that in order to form the maximum value (X) of the amount of the signal component (S) between two zero crossings (N), sampled values of the amount of the signal component (S) are supplied to a first input (20) of a comparator (21), to the second input (22) of which is supplied the content of a memory device (19), and in that the content of the memory device (19) that is current in each case is replaced by the current sampled value of the amount of the signal component (S) if the amount of the signal component (S) exceeds the value of the memory content.

3. Heart pacemaker according to claim 1 or 2, characterised in that in order to form the mean value, the maximum values (X) determined during the specified time interval are summed in an adder (30).

## Revendications

1. Stimulateur cardiaque destiné à produire un signal correspondant à la ventilation/minute d'un patient, un signal de mesure d'impédance étant relevé à l'aide d'un agencement d'électrodes (4, 10) disposé dans la région (3) du coeur et d'un dispositif de mesure d'impédance (7) raccordé, une composante de signal (S) en corrélation avec la respiration du patient étant obtenue par filtrage à partir du signal de mesure d'impédance au moyen d'un agencement de filtre (16), des passages par zéro (N) de la composante de signal (S) obtenue par filtrage étant détectés par un détecteur de passages par zéro (27), et une grandeur dépendant de la valeur absolue de la composante de signal (S) étant relevée à chaque passage par zéro (N) et utilisée pour l'élaboration en continu d'une valeur moyenne correspondant à la ventilation/minute durant un intervalle de temps prédéterminé, **caractérisé** en ce qu'en guise de grandeur dépendant de la valeur absolue de la composante de signal (S), on relève la plus grande valeur (X) sur le plan de la valeur absolue de la valeur absolue de la composante de signal (S) entre deux passages par zéro (N) respectivement successifs, et en ce que l'on forme à chaque fois la somme de deux plus grandes valeurs relevées successivement.

2. Stimulateur cardiaque selon la revendication 1, **caractérisé** en ce que pour former la valeur maximale (X) de la valeur absolue de la composante de signal (S) entre deux passages par zéro (N), on transmet des valeurs d'exploration de la valeur absolue de la composante de signal (S) à une première entrée (20) d'un comparateur (21) à la seconde entrée (22) duquel est transmis le contenu d'un dispositif de mémoire (19), et en ce que le contenu actuel respectif du dispositif de mémoire (19) est remplacé par la valeur d'exploration actuelle de la valeur absolue de la composante de signal (S) lorsque la valeur absolue de la composante de signal (S) dépasse la valeur du contenu de la mémoire.

3. Stimulateur cardiaque selon la revendication 1 ou 2, **caractérisé** en ce que pour former la valeur moyenne, on réalise, dans un additionneur (30), la somme des valeurs maximales (X) relevées durant l'intervalle de temps prédéterminé.
